# EUROPÄISCHE PATENTSCHRIFT

(11) **EP 1 654 240 B1**
(45) Veröffentlichungstag und Bekanntmachung des Hinweises auf die Patenterteilung: **02.11.2006**
(21) Anmeldenummer: 04763281.5
(22) Anmeldetag: 16.07.2004
(51) Int. Cl.: C07D 265/10

(54) **VERFAHREN ZUR HERSTELLUNG VON N-ARYL-MORPHOLINONEN**
METHOD FOR PRODUCTION OF N-ARYL MORPHOLINONES
PROCEDE DE PRODUCTION DE N-ARYL-MORPHOLINONES

(30) Priorität: 11.08.2003 DE 10336716
(43) Veröffentlichungstag der Anmeldung: 10.05.2006
(73) Patentinhaber: Merck Patent GmbH, 64293 Darmstadt (DE)
(72) Erfinder: DORSCH, Dieter, 64372 Ober-Ramstadt (DE); CEZANNE, Bertram, 64546 Mörfelden-Walldorf (DE); MEDERSKI, Werner, 64673 Zwingenberg (DE); TSAKLAKIDIS, Christos, 69469 Weinheim (DE); WURZIGER, Hanns, 64291 Darmstadt (DE)
(86) Internationale Anmeldenummer: PCT/EP2004/007938
(87) Internationale Veröffentlichungsnummer: WO 2005/016899

(56) Entgegenhaltungen:
- WO-A-02/057236
- ASTLE, M.J.; WELKS, J.D.: J. ORG. CHEM., Bd. 26, 1961, Seiten 4325-4327, XP002303446 in der Anmeldung erwähnt
- DATABASE CAPLUS [Online] CHEMICAL ABSTRACTS SERVICE, COLUMBUS, OHIO, US; TULYAGANOV, S. R. ET AL: "Reaction of some N-(2-hydroxyalkyl)arylamines with monochloroacetic acid derivatives" retrieved from STN Database accession no. 1970:466523 XP002303444 & ZHURNAL ORGANICHESKOI KHIMII , 6(6), 1305-8 CODEN: ZORKAE; ISSN: 0514-7492, 1970,

## Beschreibung

Gegenstand der Erfindung ist ein Verfahren zur Herstellung von Verbindungen der Formel I 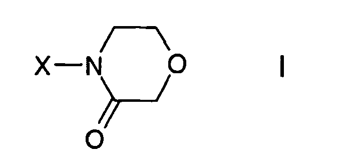 worin
X 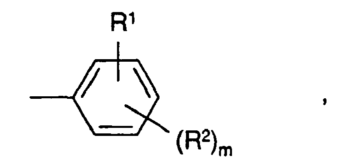
- R¹: NO₂, CN, COOR³, CON(R³)₂, COR³, SO₂R⁴, SO₂N(R³)₂, CF₃, F oder Cl,
- R²: H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-Cycloalkyl, COR³, SO₂N(R³)₂ oder SO₂R⁴,
- R³: H, A, -[C(R⁵)₂]ₙ-Ar oder -[C(R⁵)₂]ₙ-Het,
- R⁴: A, -[C(R⁵)₂]ₙ-Ar oder -[C(R⁵)₂]ₙ-Het,
- R⁵: H oder A',
- Ar: unsubstituiertes oder ein-, zwei- oder dreifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂ oder S(O)ₙA substituiertes Phenyl,
- Het: einen unsubstituierten oder ein- oder zweifach durch Hal, A, C)R⁵, N(R⁵)₂, NO₂, CN, COOR⁵, CON(R⁵)₂, NR⁵COA, NR⁵SO₂A, COR⁵, SO₂N(R⁵)₂, S(O)ₙA und/oder Carbonylsauerstoff (=O) substituierten ein- oder zweikernigen gesättigten, ungesättigten oder aromatischen Heterocyclus mit 1 bis 4 N-, O- und/oder S-Atomen,
- A': unverzweigtes oder verzweigtes Alkyl mit 1-6 C-Atomen,
- A: unverzweigtes, verzweigtes oder cylisches Alkyl mit 1-12 C- Atomen, worin eine oder zwei CH₂-Gruppen durch O- oder S- Atome und/oder durch -CH=CH-Gruppen und/oder auch 1-7 H- Atome durch F ersetzt sein können,
- Hal: F, Cl, Br oder I,
- n: 0, 1 oder 2,
- m: 0, 1, 2, 3 oder 4,
bedeuten,
sowie ihrer Salze, dadurch gekennzeichnet, daß man
a) eine Verbindung der Formel II

   X-NH₂ II

   worin
   X die oben angegebene Bedeutung hat,
   mit 5-Chlor-2,3-dihydro-[1,4]dioxin 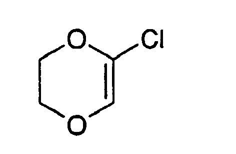 zu einer Verbindung der Formel III 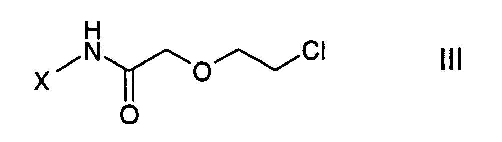 worin
   X die oben angegebene Bedeutung hat,
   umsetzt,
b) dann eine Verbindung der Formel III zu einer Verbindung der Formel I cyclisiert,
   und
c) diese gegebenenfalls in ihr Salz überführt,
   indem man eine Base oder Säure der Formel I in eines ihrer Salze umwandelt.

Der Erfindung lag die Aufgabe zugrunde, neue verbesserte Verfahren zur Herstellung von Vorstufen zu Faktor Xa-Inhibitoren aufzufinden.
Im Vergleich zu bekannten Verfahren aus dem Stand der Technik ist das erfindungsgemäße Verfahren kürzer und effizienter.

Faktor Xa Inhibitoren können zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.
Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.
Die Messung der Inhibierung von Thrombin kann z.B. nach der Methode von G. F. Cousins et al. in *Circulation* **1996,** 94, 1705-1712 erfolgen.

Eine Inhibierung des Faktors Xa kann somit verhindern, daß Thrombin gebildet wird.
Die Inhibierung des Faktors Xa und die Messung der antikoagulierenden und antithrombotischen Aktivität kann nach üblichen in vitro- oder in vivo-Methoden ermittelt werden. Ein geeignetes Verfahren wird z.B. von J.

Hauptmann et al. in *Thrombosis and Haemostasis* **1990,** *63,* 220-223 beschrieben.

Die Messung der Inhibierung von Faktor Xa kann z.B. nach der Methode von T. Hara et al. in *Thromb. Haemostas.* **1994,** *71,* 314-319 erfolgen.

Der Gerinnungsfaktor VIIa initiiert nach Bindung an Tissue Faktor den extrinsischen Teil der Gerinnungskaskade und trägt zur Aktivierung des Faktors X zu Faktor Xa bei. Eine Inhibierung von Faktor VIIa verhindert somit die Entstehung des Faktors Xa und damit eine nachfolgende Thrombinbildung.
Die Inhibierung des Faktors VIIa und die Messung der antikoagulierenden und antithrombotischen Aktivität kann nach üblichen in vitro- oder in vivo-Methoden ermittelt werden. Ein übliches Verfahren zur Messung der Inhibierung von Faktor Vlla wird z.B. von H. F. Ronning et al. in *Thrombosis Research* **1996**, 84, 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.
Die Inhibierung von Faktor IXa und die Messung der antikoagulierenden und antithrombotischen Aktivität kann nach üblichen in vitro- oder in vivo-Methoden ermittelt werden. Ein geeignetes Verfahren wird z.B. von J. Chang et al. in *Journal of Biological Chemistry* **1998,** *273,* 12089-12094 beschrieben.

Ein Zusammenhang zwischen dem Tissuefaktor TF / Faktor VIIa und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt. Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92.

In WO 02/057236 sind andere Verfahren und Morpholinon-Vorstufen beschrieben.

Zur Herstellung von 2-(2-Chlorethoxy)-acetamiden sind in der Literatur folgende Methoden bekannt:

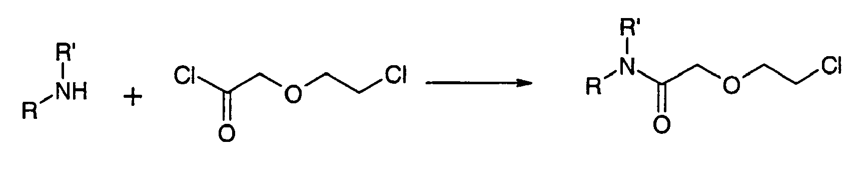

Diese Methode ist z. B. beschrieben in US 3 074 939, BE 776767 und DE 1922613.

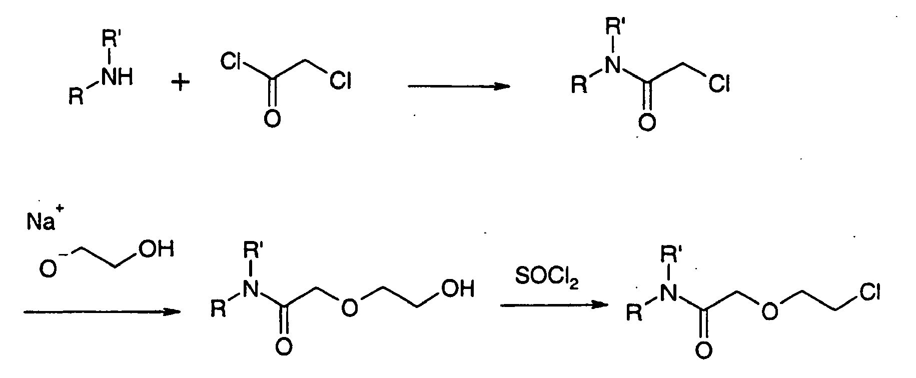

Diese Methode ist z. B. beschrieben in G. May, D. Peteri, Arzneim.-Forsch. (Drug Res.) 23, 718 (1973).

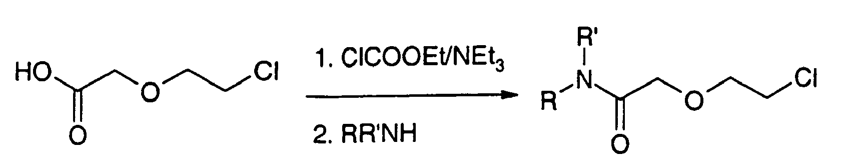

Diese Methode ist z. B. beschrieben in DE 2150075.

Diese Methoden besitzen jedoch Nachteile. So sind viele Reaktionsstufen nötig oder die Ausgangsmaterialien sind teuer.

Von M. J. Astle, J. D. Welks, J. Org. Chem. 26, 4325 (1961) ist folgende Reaktion bekannt:

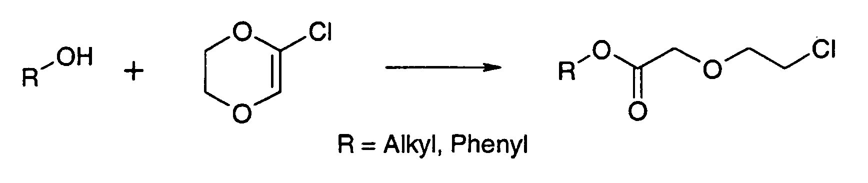

Wir haben überraschend gefunden, dass auch Arylamine, sofern sie einen pKₐ kleiner oder gleich 3 besitzen, mit 2-Chlordioxen zu 2-(2-Chlorethoxy)-acetamiden reagieren.

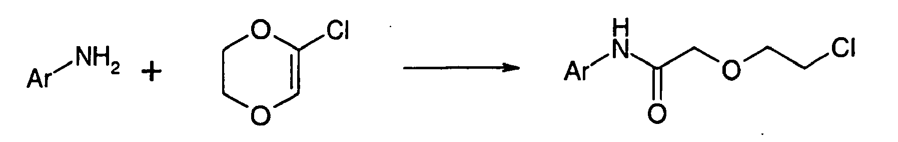

Im Hinblick auf M. J. Astle, J. D. Welks, J. Org. Chem. 26, 4325 (1961) ist dies unerwartet, da Amine wie Ammoniak, Benzylamin, 8-Aminochinolin oder 4-Methoxyanilin nicht oder sehr schlecht reagieren.

Vergleich von pKₐ-Werten:

| | |
|---|---|
| Benzylamin | 9.5 |
| Ammoniak | 9.24 |

8-Aminochinolin 0.7 (NH2-Gruppe) und 4.0 (Chinolinstickstoff). Der basische Chinolinstickstoff verhindert die Reaktion.

| | |
|---|---|
| 4-Methoxyanilin | 5.4 |
| 4-Nitroanilin | 1.0 |
| 4-Cyanoanilin | 1.7 |
| 3-Nitroanilin | 2.5 |
| 2-Methyl-4-nitroanilin | 1.04 |
| Methyl-4-aminobenzoat | 1.5 |
| 4-Aminobenzophenon | 2.2 |
| 2-Nitroanilin | -0.23 |

Vorteilhaft ist bei der Umsetzung ein Zusatz einer Säure, z. B. einer Brönsted-Säure wie Chlorwasserstoffsäure oder einer Lewis-Säure oder aber ein Zusatz von 2,2-Dichlordioxen, einer Verbindung, die, wie aus der Literatur (R. K. Summerbell, H. E. Lunk, J. Am. Chem. Soc. 79, S. 4802, 1957) bekannt ist, leicht in Chlorwasserstoff und 2-Chlordioxen dissoziiert. Die Reaktion kann in vielen Lösungsmitteln, z. B. Toluol, Acetonitril, Dioxan, aber auch in Substanz, also ohne Lösungsmittel durchgeführt werden. Typische Reaktionstemperaturen sind 0 bis 150 °C, in der Regel um 80 °C, z.B. zwischen 70 und 90 °C.

Der Vorteil dieses Verfahrens liegt in der leichten Zugänglichkeit von 2-Chlordioxen bzw. 2,2-Dichlordioxan.

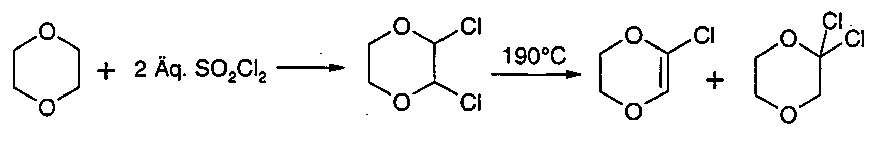

Die Herstellung von 2,3-Dichlordioxan ist z. B. beschrieben in M. Iyoda et al, Heterocycles, 54, S. 833, 2001. Die thermische Eliminierung von Chlorwasserstoff ist beschrieben in US 2 756 240. Bei dieser Methode erhält man 2-Chlordioxen, das mit einem gewissen Anteil 2,2-Dichlordioxan (typischerweise 5 bis 50%) verunreinigt ist.

Von N. V. Kuznetsov, I. I. Krasavtsev, Sov. Prog. Chem. (Engl. Transl.) 44, S. 77, 1987 sind Methoden zur Herstellung von 2-Chlordioxen aus 2,3-Dichlordioxan mit Natriumhydroxid beschrieben.

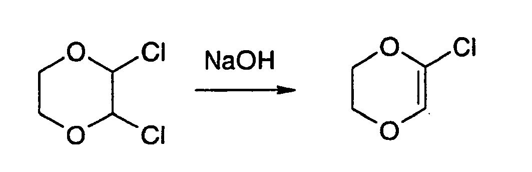

Die Cyclisierung von Chlorethoxyacetamiden zu Morpholinonen wurde bislang nur in zwei Veröffentlichungen beschrieben, in DE 922613 und L. Fumagalli et al. Pharmazie 30, 78 (1975).
In beiden Fällen handelt es sich um Trijodbenzoesäure- und Trijodphenylalkansäurederivate.

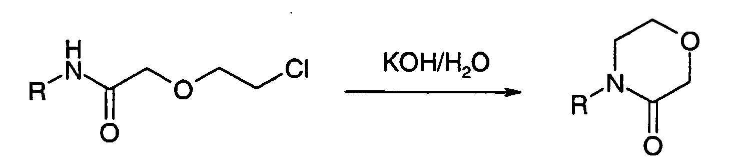

Dieses Verfahren eignet sich jedoch nur für Substrate, die wasserlöslich sind, wie in den obigen Dokumenten, in denen R stets eine freie Carbonsäuregruppe enthält.

Wir haben gefunden, dass sich Chlorethoxyacetamide vorzugsweise mit schwachen Basen wie z.B. Caesiumcarbonat oder Kaliumcarbonat in einem geeigneten Lösungsmittel wie z.B. Acetonitril zu Morpholinonen cyclisieren lassen.

Vor- und nachstehend bedeutet A Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2-, 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl oder Trifluormethyl.

A' bedeutet bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl oder Trifluormethyl.

Cycloalkyl hat 3-7 C-Atome und bedeutet vorzugsweise Cyclopropyl, Cyclobutyl, Cyclopentyl oder Cylohexyl.

Hal bedeutet vorzugsweise F, Cl oder Br, aber auch I.
R¹ bedeutet vorzugsweise NO₂, CN, COOH, COOR³ COR³ oder Cl.
R² bedeutet vorzugsweise H, Hal oder A.
R³ bedeutet vorzugsweise H, A' oder -[C(R⁵)₂]ₙ-Ar.
R⁴ bedeutet vorzugsweise A.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet vorzugsweise z.B. unsubstituiertes oder ein-, zwei- oder dreifach durch Hal, A, OR⁵, SO₂A, COOR⁵ oder CN substituiertes Phenyl. Ar bedeutet insbesondere bevorzugt z.B. unsubstituiertes oder ein- oder zweifach durch Hal, A, OA, SO₂A, SO₂NH₂, COOR⁵ oder CN substituiertes Phenyl, wie z.B. Phenyl, 2-Methylsulfonylphenyl, 2-Aminosulfonylphenyl, 2-, 3- oder 4-Chlorphenyl, 4-Methylphenyl, 4-Bromphenyl, 3-Fluor-4-methoxyphenyl, 4-Trifluormethoxyphenyl, 4-Ethoxyphenyl, 2-Methoxyphenyl, 3-Cyanphenyl oder 4-Ethoxycarbonylphenyl.
Ganz besonders bevorzugt bedeutet Ar unsubstituiertes Phenyl.

Het ist unsubstituiert oder ein- oder zweifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA und/oder Carbonylsauerstoff (=O) substituiert und bedeutet z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5- Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.
n bedeutet vorzugsweise 0 oder 1.
m bedeutet vorzugsweise 0, 1 oder 2.

Gegenstand der Erfindung ist vorzugsweise ein Verfahren nach Anspruch 1, zur Herstellung von Verbindungen der Formel I, worin
- R¹: NO₂, CN, COOR³, COR³ oder Cl,
- R²: H, Hal oder A bedeuten.

Bevorzugt ist weiterhin ein Verfahren nach Anspruch 1 oder 2, zur Herstellung von Verbindungen der Formel 1, worin
- R¹: NO₂, CN, COOR³, CON(R³)₂, COR³, SO₂R⁴, SO₂N(R³)₂, CF₃, F oder Cl,
- R²: H, Hal oder A,
- R³: H, A, -[C(R⁵)₂]ₙ-Ar oder -[C(R⁵)₂]ₙ-Het bedeuten.

Weiterhin bevorzugt ist ein Verfahren nach Anspruch 1 zur Herstellung von Verbindungen der Formel I, worin Ar Phenyl bedeutet.

Weiterhin bevorzugt ist ein Verfahren zur Herstellung von Verbindungen der Formel I, worin R⁴ A bedeutet.

Weiter ist bevorzugt ein Verfahren zur Herstellung von Verbindungen der Formel I, worin
- R¹: NO₂, CN, COOR³, CON(R³)₂, COR³, CF₃, F oder Cl,
- R²: H, Hal oder A',
- R³: H, A' oder -[C(R⁵)₂]ₙ-Ar,
- Ar: Phenyl,
- R⁵: H oder A',
- A': unverzweigtes oder verzweigtes Alkyl mit 1-6 C-Atomen,
- Hal: F, Cl, Br oder I,
- n: 0, 1 oder 2,
bedeuten.

Ganz besonders bevorzugt ist ein Verfahren nach Anspruch 1 zur Herstellung von Verbindungen ausgewählt aus der Gruppe
4-(4-Nitrophenyl)-3-oxo-morpholin,
4-(3-Nitrophenyl)-3-oxo-morpholin,
4-(2-Nitrophenyl)-3-oxo-morpholin,
2-Methyl-4-(4-nitrophenyl)-3-oxo-morpholin,
4-(4-Methoxycarbonylphenyl)-3-oxo-morpholin,
4-(4-Benzoylphenyl)-3-oxo-morpholin.

Weiterhin bevorzugt ist ein Verfahren nach einem oder mehreren der Ansprüche 1-6, zur Herstellung von Verbindungen der Formel I, wobei das Amin der Formel II einen pKₐ-Wert ≤ 3 aufweist.

Die Verbindungen der Formel I können vorzugsweise erhalten werden, indem man in einem ersten Schritt a) Verbindungen der Formel II mit 5-Chlor-2,3-dihydro-[1,4]dioxin zu einer Verbindung der Formel III umsetzt. Die Umsetzung erfolgt in der Regel in einem inerten Lösungsmittel, sie kann aber auch ohne Lösungsmittel in Substanz durchgeführt werden. Vorteilhaft ist ein Zusatz einer Säure, z. B. einer Brönsted-Säure wie Chlorwasserstoffsäure oder einer Lewis-Säure oder aber ein Zusatz von 2,2-Dichlordioxen, einer Verbindung, die, wie aus der Literatur (R. K. Summerbell, H. E. Lunk, J. Am. Chem. Soc. 79, S. 4802, 1957) bekannt ist, leicht in Chlorwasserstoff und 2-Chlordioxen dissoziiert.
Die Reaktionszeit liegt je nach den angewendeten Bedingungen zwischen einigen Minuten und 14 Tagen, vorzugsweise zwischen einer und zehn Stunden, die Reaktionstemperatur zwischen etwa 0° und 150°, normalerweise zwischen 20° und 130°, vorzugsweise zwischen 60° und 110°, ganz besonders bevorzugt zwischen 70° und 90° C.

Als inerte Lösungsmittel eignen sich z.B. Wasser; Kohlenwasserstoffe wie Hexan, Petrolether, Benzol, Toluol oder Xylol; chlorierte Kohlenwasserstoffe wie Trichlorethylen, 1,2-Dichtorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Acetonitril.

In einem zweiten Schritt b) werden Verbindungen der Formel III zu den Verbindungen der Formel I cyclisiert.
Die Umsetzung erfolgt in der Regel in einem inerten Lösungsmittel, vorzugsweise in Gegenwart eines Alkali- oder Erdalkalimetall-hydroxids, -carbonats oder -bicarbonats. Ganz besonders bevorzugt sind schwache Basen wie Cäsium- oder Kaliumcarbonat.

Die Reaktionszeit liegt je nach den angewendeten Bedingungen zwischen einigen Minuten und 14 Tagen, vorzugsweise zwischen einer und zwanzig Stunden, die Reaktionstemperatur zwischen etwa 0° und 150°, normalerweise zwischen 0° und 90°, vorzugsweise zwischen 10° und 70°, besonders bevorzugt zwischen 20° und 50° C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel, besonders bevorzugt ist Acetonitril.

Die Verfahrensschritte a) und b) können auch als Eintopfreaktion durchgeführt werden.
Nachdem sich Amin und 2-Chlordioxen vollständig umgesetzt haben, senkt man die Temperatur der Lösung und gibt einen Überschuss Alkalicarbonat (typischerweise 1,5 bis 4 Äquivalente) zu und rührt das Reaktionsgemisch bis zum vollständigen Umsatz.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Gegenstand der Erfindung sind auch die Zwischenverbindungen der Formel III 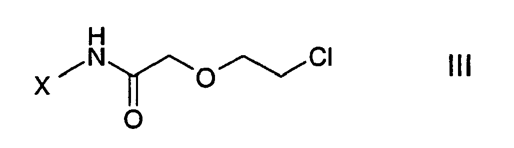 worin
X 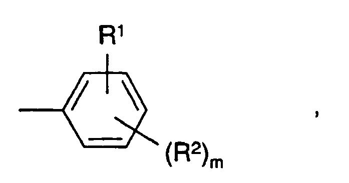
- R¹: NO₂ oder CN,
- R²: H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-Cycloalkyl, COR³, SO₂N(R³)₂ oder SO₂R⁴,
- R³: H, A, -[C(R⁵)₂]ₙ-Ar oder -[C(R⁵)₂]ₙ-Het,
- R⁴: A, -[C(R⁵)₂]ₙ-Ar oder -[C(R⁵)₂]ₙ-Het,
- R⁵: H oder A',
- Ar: unsubstituiertes oder ein-, zwei- oder dreifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂ oder S(O)ₙA substituiertes Phenyl,
- Het: einen unsubstituierten oder ein- oder zweifach durch Hal, A, OR⁵, N(R⁵)₂, NO₂, CN, COOR⁵, CON(R⁵)₂, NR⁵COA, NR⁵SO₂A, COR⁵, S0₂N(R⁵)₂, S(O)ₙA und/oder Carbonylsauerstoff (=O) substituierten ein- oder zweikernigen gesättigten, ungesättigten oder aromatischen Heterocyclus mit 1 bis 4 N-, O- und/oder S-Atomen,
- A': unverzweigtes oder verzweigtes Alkyl mit 1-6 C-Atomen,
- A: unverzweigtes, verzweigtes oder cylisches Alkyl mit 1-12 C- Atomen, worin eine oder zwei CH₂-Gruppen durch O- oder S- Atome und/oder durch -CH=CH-Gruppen und/oder auch 1-7 H- Atome durch F ersetzt sein können,
- Hal: F, Cl, Br oder I,
- n: 0, 1 oder 2,
- m: 0, 1, 2, 3 oder 4,
bedeuten,
sowie ihre Salze.

Die Zwischenverbindungen sind wichtig zur Herstellung der Verbindungen der Formel I.
Die bevorzugten Bedeutungen der Reste entsprechen denen wie oben ausgeführt, falls nicht ausdrücklich etwas anderes angegeben ist.

Gegenstand der Erfindung sind auch die Zwischenverbindungen nach Anspruch 15, worin
- R¹: NO₂ oder CN,
- R²: H, Hal oder A,
bedeuten,
sowie ihre Salze.

Bevorzugt sind weiterhin Zwischenverbindungen nach Anspruch 15, worin
- R¹: NO₂ oder CN,
- R²: H, Hal oder A,
- R³: H, A, -[C(R⁵)₂]ₙ-Ar oder -[C(R⁵)₂]ₙ-Het,
bedeuten,
sowie ihre Salze.

Weiterhin bevorzugt sind Zwischenverbindungen nach Anspruch 15, 16 oder 17, worin
- Ar: Phenyl bedeutet,
sowie ihre Salze.

Bevorzugt sind weiterhin Zwischenverbindungen nach einem oder mehreren der Ansprüche 15-18, worin
- R⁴: A bedeutet,
sowie ihrer Salze.

Besonders bevorzugt sind Zwischenverbindungen nach einem oder mehreren der Ansprüche 15-19, worin
- R¹: NO₂ oder CN,
- R²: H, Hal oder A',
- R³: H, A' oder -[C(R⁵)₂]ₙ-Ar,
- Ar: Phenyl,
- R⁵: H oder A',
- A': unverzweigtes oder verzweigtes Alkyl mit 1-6 C-Atomen,
- Hal: F, Cl, Br oder I,
- n: 0, 1 oder 2,
- m: 0, 1 oder 2,
bedeuten,
sowie ihre Salze.

Besonders bevorzugt sind Zwischenverbindungen nach Anspruch 20,
worin
- R¹: NO₂,
- R²: H, Hal oder A',
- R³: H, A' oder -[C(R⁵)₂]ₙ-Ar,
- Ar: Phenyl,
- R⁵: H oder A',
- A': unverzweigtes oder verzweigtes Alkyl mit 1-6 C-Atomen,
- Hal: F, Cl, Br oder I,
- n: 0, 1 oder 2,
- m: 0, 1 oder 2,
bedeuten,
sowie ihre Salze.

Gegenstand der Erfindung ist auch ein Verfahren zur Herstellung von Zwischenverbindungen der Formel III, 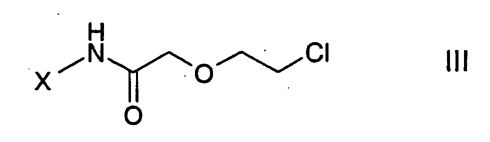 worin
X 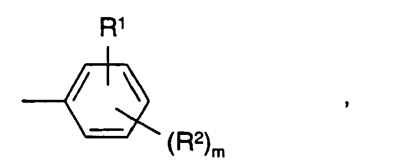
- R¹: NO₂, CN, COOR³, CON(R³)₂, COR³, SO₂R⁴, SO₂N(R³)₂, CF₃, F oder Cl,
- R²: H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-Cycloalkyl, COR³, SO₂N(R³)₂ oder SO₂R⁴,
- R³: H, A, -[C(R⁵)₂]ₙ-Ar oder -[C(R⁵)₂]ₙ-Het,
- R⁴: A, -[C(R⁵)₂]ₙ-Ar oder -[C(R⁵)₂]ₙ-Het,
- R⁵: H oder A',
- Ar: unsubstituiertes oder ein-, zwei- oder dreifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂ oder S(O)ₙA substituiertes Phenyl,
- Het: einen unsubstituierten oder ein- oder zweifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA und/oder Carbonylsauerstoff (=O) substituierten ein- oder zweikernigen gesättigten, ungesättigten oder aromatischen Heterocyclus mit 1 bis 4 N-, O- und/oder S-Atomen,
- A': unverzweigtes oder verzweigtes Alkyl mit 1-6 C-Atomen,
- A: unverzweigtes, verzweigtes oder cylisches Alkyl mit 1-12 C- Atomen, worin eine oder zwei CH₂-Gruppen durch O- oder S- Atome und/oder durch -CH=CH-Gruppen und/oder auch 1-7 H- Atome durch F ersetzt sein können,
- Hal: F, Cl, Br oder I,
- n: 0, 1 oder 2,
- m: 0, 1, 2, 3 oder 4,
bedeuten,
sowie ihrer Salze, dadurch gekennzeichnet, daß man
a) eine Verbindung der Formel II

   X-NH₂ II

   worin
   X die oben angegebene Bedeutung hat,
   mit 5-Chlor-2,3-dihydro-[1,4]dioxin 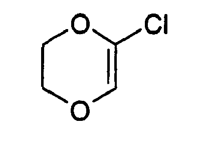 umsetzt,
   und
   gegebenenfalls die Verbindung der Formel III in ihr Salz überführt.

Die Bedingungen des Verfahrens, insbesondere die bevorzugten sind die gleichen wie unter dem Verfahren zur Herstellung der Verbindung der Formel I angegeben.
Die bevorzugten Bedeutungen der Reste entsprechen denen wie oben ausgeführt, falls nicht ausdrücklich etwas anderes angegeben ist.

Bevorzugt ist ein Verfahren nach Anspruch 22 zur Herstellung von Zwischenverbindungen der Formel III,
worin
- R¹: NO₂ oder CN,
- R²: H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-Cycloalkyl, COR³, SO₂N(R³)₂ oder SO₂R⁴,
- R³: H, A, -[C(R⁵)₂]ₙ-Ar oder -[C(R⁵)₂]ₙ-Het,
- R⁴: A, -[C(R⁵)₂]ₙ-Ar oder -[C(R⁵)₂]ₙ-Het,
- R⁵: H oder A',
- Ar: unsubstituiertes oder ein-, zwei- oder dreifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂ oder S(O)ₙA substituiertes Phenyl,
- Het: einen unsubstituierten oder ein- oder zweifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA und/oder Carbonylsauerstoff (=O) substituierten ein- oder zweikernigen gesättigten, ungesättigten oder aromatischen Heterocyclus mit 1 bis 4 N-, O- und/oder S-Atomen,
- A': unverzweigtes oder verzweigtes Alkyl mit 1-6 C-Atomen,
- A: unverzweigtes, verzweigtes oder cylisches Alkyl mit 1-12 C- Atomen, worin eine oder zwei CH₂-Gruppen durch O- oder S- Atome und/oder durch -CH=CH-Gruppen und/oder auch 1-7 H- Atome durch F ersetzt sein können,
- Hal: F, Cl, Br oder I,
- n: 0, 1 oder 2,
- m: 0, 1, 2, 3 oder 4,
bedeuten.

Weiter bevorzugt ist ein Verfahren nach Anspruch 23 zur Herstellung von Zwischenverbindungen der Formel III,
worin
- R¹: NO₂ oder CN,
- R²: H, Hal oder A,
bedeuten.

Weiter bevorzugt ist ein Verfahren nach Anspruch 23 zur Herstellung von Zwischenverbindungen der Formel III,
worin
- R¹: NO₂ oder CN,
- R²: H, Hal oder A,
- R³: H, A, -[C(R⁵)₂]ₙ-Ar oder -[C(R⁵)₂]ₙ-Het,
bedeuten.

Weiter bevorzugt ist ein Verfahren nach Anspruch 23 zur Herstellung von Zwischenverbindungen der Formel III,
worin
- Ar: Phenyl bedeutet.

Bevorzugt ist auch ein Verfahren nach Anspruch 23 zur Herstellung von Zwischenverbindungen der Formel III,
worin
- R⁴: A bedeutet.

Besonders bevorzugt ist ein Verfahren nach Anspruch 23 zur Herstellung von Zwischenverbindungen der Formel III,
worin
- R¹: NO₂ oder CN,
- R²: H, Hal oder A',
- R³: H, A' oder -[C(R⁵)₂]ₙ-Ar,
- Ar: Phenyl,
- R⁵: H oder A',
- A': unverzweigtes oder verzweigtes Alkyl mit 1-6 C-Atomen,
- Hal: F, Cl, Br oder I,
- n: 0, 1 oder 2,
- m: 0, 1 oder 2,
bedeuten.

Vor- und nachstehend sind alle Temperaturen in °C angegeben. Massenspektrometrie (MS): EI (Elektronenstoß-Ionisation) M⁺;
ESI (Electrospray lonization) (M+H)⁺;
FAB (Fast Atom Bombardment) (M+H)⁺

### Beispiel 1

### 4-(4-Nitrophenyl)-3-oxo-mornholin

Die Herstellung erfolgt analog nachstehendem Schema:

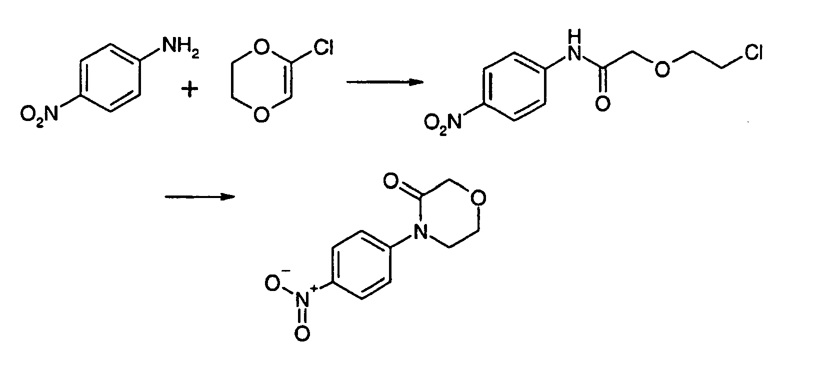

### 1.1 ohne Lösungsmittel:

1.00 g (7.24 mmol) 4-Nitroanilin wird mit 1.53 g eines Gemischs von 2-Chlordioxen und 2,2-Dichlordioxan (Molverhältnis 1 : 1) versetzt und die Mischung unter Rühren auf 80°C erhitzt. Es bildet sich innerhalb einer Stunde eine feste braune Masse, die wieder flüssig wird und innerhalb der folgenden 12 Stunden kristallisiert. Das Rohprodukt wird aus Ethanol unter Zusatz von Wasser umkristallisiert. Man erhält 1.80 g 2-(2-Chlorethoxy)-N-(4-nitro-phenyl)-acetamid ("A1") als gelbliche Kristalle, F. 101-102°C. ¹H-NMR (d⁶-DMSO): δ = 3.82 (m; 4H), 4.23 (s; 2H), 7.91 (d, J = 9 Hz, 2H), 8.23 (d, J = 9 Hz, 2H), 10.34 (s, 1 H).

### 1.2 in Acetonitril:

Eine Lösung von 276 mg (2.00 mmol) 4-Nitroanilin in 2 ml Acetonitril wird mit 310 mg eines Gemischs von 2-Chlordioxen und 2,2-Dichlordioxan (Molverhältnis 1 :1) versetzt und die Lösung unter Rühren 18 Stunden auf 80° C erhitzt. Das Reaktionsgemisch wird eingedampft und der Rückstand aus Ethanol/Wasser umkristallisiert: 360 mg "A1" als gelbliche Kristalle.

### 1.3 1 kg "A1" wird bei Raumtemperatur in 5 Liter Acetonitril gelöst, mit 835 g Kaliumcarbonat versetzt und 18 Stunden bei dieser Temperatur gerührt. Man erwärmt auf 50°, arbeitet analog Beispiel 6 auf und erhält 4-(4-Nitrophenyl)-3-oxo-morpholin ("A2"), F. 150-152°.

### Beispiel 2

### 4-(4-Nitro-2-methyl-phenyl)-3-oxo-morpholin

Eine Lösung von 1.10 g (7.24 mmol) 2-Methyl-4-nitroanilin in 20 ml THF wird mit 1.05 g eines Gemischs von 2-Chlordioxen und 2,2-Dichlordioxan (Molverhältnis 1 : 1) versetzt und zum Sieden erhitzt. Das Lösungsmittel wird abdestilliert und der Rückstand, eine braune viskose Flüssigkeit, 18 Stunden auf 80° C erhitzt. Nach Abkühlen wird der Rückstand aus Toluol/tert.Butylmethylether umkristallisiert: 1.50 g 2-(2-Chlorethoxy)-N-(2-methyl-4-nitrophenyl)-acetamid als gelbliche Kristalle, F. 113-114°C. ¹H-NMR (d⁶-OMSO): δ = 2.35 (s; 3H), 3.82 (m; 4H), 4.23 (s; 2H), 8.05 (d, J = 8Hz, 1 H) 8.09 (dd, J = 9 Hz, J = 1 Hz, 1 H), 8.16 (d, J = 1 Hz, 1 H), 9.33 (s, 1H).

Die Cyclisierung erfolgt analog 1.3.
Man erhält 4-(4-Nitro-2-methyl-phenyl)-3-oxo-morpholin, ESI 237.

### Beispiel 3

### 4-(2-Nitrophenyl)-3-oxo-morpholin

1.12 g (8.12 mmol) 2-Nitroanilin wird mit 1.12 g eines Gemischs von 2-Chlordioxen und 2,2-Dichlordioxan (Molverhältnis 89 : 11) versetzt und die Mischung unter Rühren auf 80°C erhitzt. Es bildet sich eine viskose Flüssigkeit, die 3 Stunden gerührt wird. Beim Abkühlen auf Raumtemperatur kristallisiert das Produkt: 2.1 g 2-(2-Chlorethoxy-N-(2-nitrophenyl)-acetamid als gelbliche Kristalle. ¹H-NMR (d⁶-DMSO): δ = 3.84 (m; 4H), 4.25 (s; 2H), 7.35 (t, J = 8 Hz, 1 H), 7.77 (t, J = 8 Hz, 1 H), 8.14 (d, J = 8 Hz, 1 H), 8.30 (d, J = 8 Hz, 1 H), 10.74 (s, 1 H).
Die Cyclisierung erfolgt analog 1.3.
Man erhält 4-(2-Nitrophenyl)-3-oxo-morpholin, ESI 223.

### Beispiel 4

### 4-(4-Cyanehenyl)-3-oxo-moroholin

Ein Gemisch von 959 mg (8.12 mmol) 4-Aminobenzonitril und 1.12 g eines Gemischs von 2-Chlordioxen und 2,2-Dichlordioxan (Molverhältnis 89 : 11) wird unter Rühren 18 Stunden auf 80° C erhitzt. Beim Abkühlen auf Raumtemperatur kristallisiert das Produkt: 1.9 g 2-(2-Chlorethoxy)-N-(4-cyanphenyl)-acetamid als gelbliche Kristalle. ¹H-NMR (d⁶-DMSO): δ = 3.82 (m; 4H), 4.19 (s; 2H), 7.78 (d, J = 8 Hz, 2H), 7.85 (d, J = 8 Hz, 2H), 10.22 (s, 1H).

Die Cyclisierung erfolgt analog 1.3.
Man erhält 4-(4-Cyanphenyl)-3-oxo-morpholin, ESI 203.

### Beispiel 5

### 4-(4-Methoxycarbonylphenyl)-3-oxo-moroholin

Ein Gemisch von 1.23 mg (8.12 mmol) Methyl-4-aminobenzoat und 1.12 g eines Gemischs von 2-Chlordioxen und 2,2-Dichlordioxan (Molverhältnis 89 : 11) wird unter Rühren 18 Stunden auf 80° C erhitzt. Beim Abkühlen auf Raumtemperatur kristallisiert das Produkt: 2.2 g 4-[2-(2-Chlorethoxy)-acetylamino]-benzoesäuremethylester als gelbliche Kristalle. ¹H-NMR (d⁶-OMSO): δ = 3.82 (m; 7H), 4.20 (s; 2H), 7.82 (d, J = 8 Hz, 2H), 7.93 (d, J = 8 Hz, 2H), 10.15 (s, 1H).

Die Cyclisierung erfolgt analog 1.3.
Man erhält 4-(4-Methoxycarbonyl-phenyl)-3-oxo-morpholin, ESI 236.

### Beispiel 6

### Eintopfreaktion zur Herstellung von "A2"

Eine Lösung von 6.00 g (24.9 mmol) 4-Nitroanilin in 40 ml Acetonitril wird mit 6.40 g 2-Chlordioxen (enthält 6% 2,2-Dichlordioxan) versetzt und 18 Stunden bei 80° C gerührt. Die Reaktionslösung wird auf 40° C abgekühlt, mit 18.0 g (130 mmol) Kaliumcarbonat versetzt und 14 Stunden bei dieser Temperatur gerührt. Das Reaktionsgemisch wird filtriert, der Rückstand gut mit Acetonitril gewaschen und das Filtrat eingedampft. Der Rückstand wird aus Acetonitril umkristallisiert: 8.2 g bräunliche Kristalle ("A2"), F. 150-152°C. ¹H-NMR (d⁶-DMSO): δ = 3.86 (t, J = 5 Hz; 2H), 4.02 (t, J = 5 Hz; 2H), 4.28 (s; 2H), 7.77 (d, J = 9 Hz, 2H), 8.28 (d, J = 9 Hz, 2H).

### Beispiel 7

### 4-(3-Nitrophenyl)-3-oxo-moroholin

Ein Gemisch von 1.12 g (8.12 mmol) 3-Nitroanilin und 1.11 g 2-Chlordioxen (enthält 6% 2,2-Dichlordioxan) wird unter Rühren 24 Stunden auf 80° C erhitzt. Man erhält 2.1 g 2-(2-Chlorethoxy)-N-(3-nitrophenyl)-acetamid als braunes ÖI. ESI 259.

Die Cyclisierung erfolgt analog 1.3.
Man erhält 4-(3-Nitrophenyl)-3-oxo-morpholin, ESI 223.

### Beispiel 8

### 4-(4-Benzoyl-phenyl)-3-oxo-morpholin

Ein Gemisch von 1.60 g (8.12 mmol) 4-Aminobenzophenon und 1.11 g 2-Chlordioxen (enthält 6% 2,2-Dichlordioxan) wird unter Rühren 24 Stunden auf 80° C erhitzt. Man erhält 2.6 g N-(4-Benzoylphenyl)-2-(2-chlorethoxy)-acetamid als braunes ÖI. ESI 318.

Die Cyclisierung erfolgt analog 1.3.
Man erhält 4-(4-Benzoyl-phenyl)-3-oxo-morpholin, ESI 282.

### Beispiel 9

### 4-(3-Fluor-phenyl)-3-oxo-morpholin

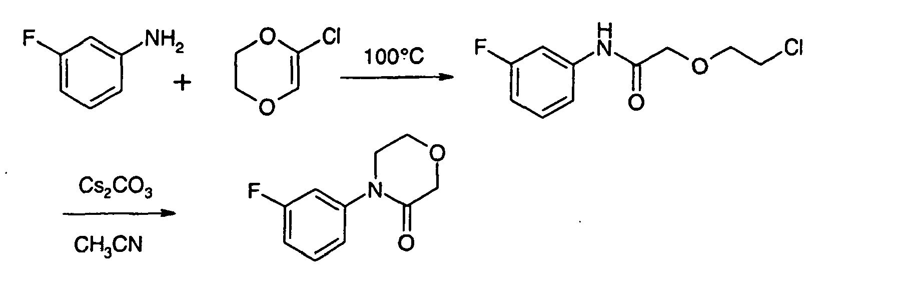

Ein Gemisch von 12.0 g (108 mmol) 3-Fluoranilin und 16 g 2-Chlordioxen (enthält 6% 2,2-Dichlordioxan) wird 24 Stunden auf 100 °C erhitzt. Man lässt abkühlen und entfernt überschüssiges 2-Chlordioxen im Vakuum. Man erhält 25 g 2-(2-Chlorethoxy)-N-(3-fluorphenyl)-acetamid als braunes Öl; ESI 232. Dieses Öl wird in 400 ml Acetonitril gelöst und mit 84.7 g (260 mmol) Caesiumcarbonat versetzt. Die entstandene Suspension wird 18 Stunden bei Raumtemperatur gerührt. Das Reaktionsgemisch wird filtriert und das Filtrat eingedampft. Man erhält 21.0 g 4-(3-Fluorphenyl)-morpholin-3-on als braunes Öl; ESI 196. ¹H-NMR (d⁶⁻DMSO): δ = 3.77 (t, J = 5 Hz; 2H), 3.97 (t, J = 5 Hz; 2H), 4.23 (s; 2H), 7.11 (dddd, J₁ = 8 Hz, J₂ = 8 Hz, J₃ = 2 Hz, J₄ = 0.5 Hz, 1 H), 7.26 (ddd, J₁ = 8 Hz, J₂ = 2 Hz, J₃ = 0.5 Hz, 1 H), 7.34 (ddd, J₁ = 10 Hz, J₂ = 2 Hz, J₃ = 2 Hz, 1 H), 7.45 (ddd, J₁ = 8 Hz, J₂ = 8 Hz, J₃ = 7 Hz, 1 H).

### Beispiel 10

### 4-(3-Methyl-4-nitro-phenyl)-3-oxo-momholin

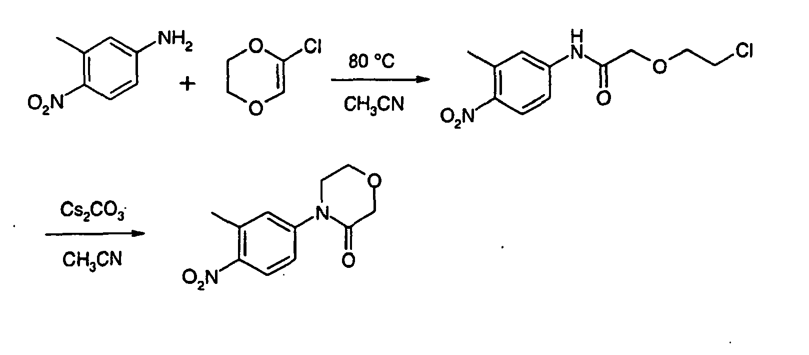

Eine Lösung von 10.0 g (65.7 mmol) 3-methyl-4-nitroanilin in 250 ml Acetonitril wird mit 12.8 g 2-Chlordioxen (enthält 6% 2,2-Dichlordioxan) versetzt und 66 Stunden bei 80° C gerührt. Die Reaktionslösung wird auf Raumtemperatur abgekühlt, mit 42.8 g (131 mmol) Caesiumcarbonat versetzt und 18 Stunden bei Raumtemperatur gerührt. Das Reaktionsgemisch wird filtriert, der Rückstand gut mit Acetonitril gewaschen und das Filtrat eingedampft. Der Rückstand wird aus wenig Acetonitril umkristallisiert. Man erhält 12.8 g (83%) 4-(3-Methyl-4-nitrophenyl)-morpholin-3-on als gelblicher Feststoff. ESI 236. ¹H-NMR (d⁶-DMSO): δ = 2.54 (s; 2H), δ = 3.82 (t, J = 5 Hz; 2H), 4.00 (t, J = 5 Hz; 2H), 4.25 (s; 2H), 7.57 (m; 2H), 8.04 (d, J.= 8 Hz; 1 H).

Analog erhält man 4-(2-Chlor-5-fluor-4-nitro-phenyl)-3-oxo-morpholin

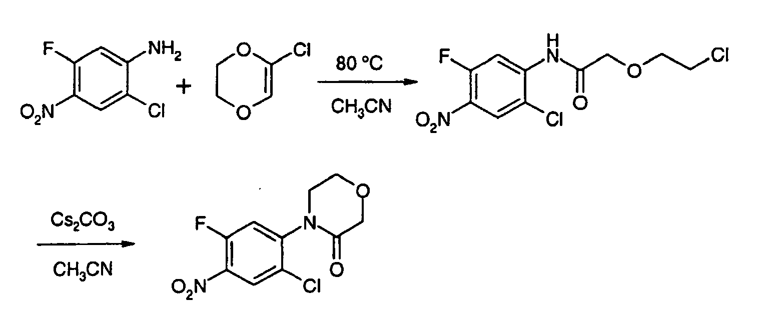

### Beispiel 11

Analog Beispiel 7 erhält man 4-(2-Brom-5-nitro-phenyl)-3-oxo-morpholin

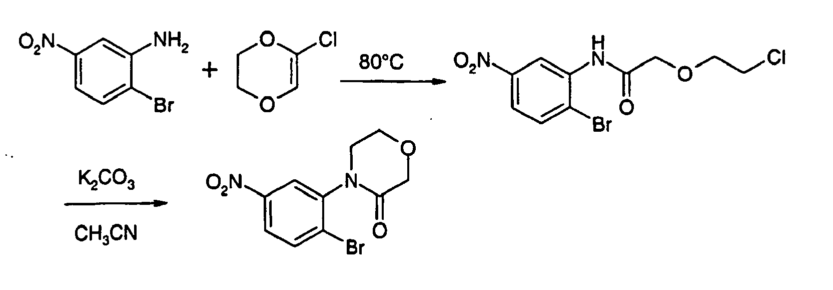

Analog Beispiel 7 erhält man 4-(2-Methoxycarbonyl-5-nitro-phenyl)-3-oxomorpholin

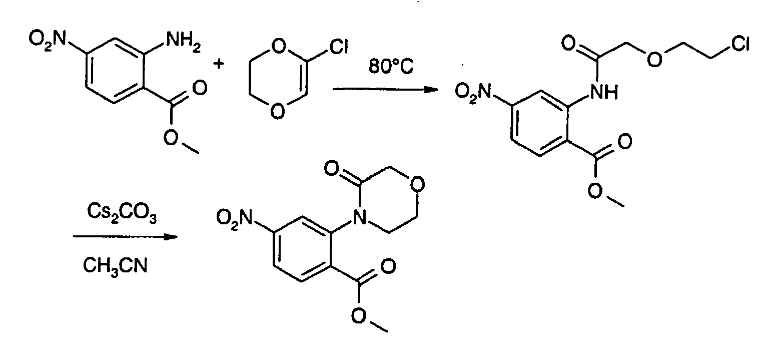

## Patentansprüche

1. Verfahren zur Herstellung von Verbindungen der Formel I 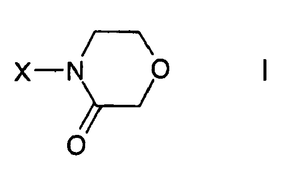 worin
x 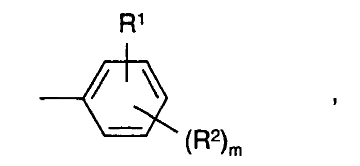
R¹ NO₂, CN, COOR³, CON(R³)₂, COR³, SO₂R⁴, SO₂N(R³)₂, CF₃, F oder Cl,
R² H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -(C(R⁵)₂]ₙ-Cycloalkyl, COR³, SO₂N(R³)₂ oder SO₂R⁴,
R³ H, A, -[C(R⁵)₂]ₙ-Ar oder -[C(R⁵)₂]ₙ-Het,
R⁴ A, -[C(R⁵)₂]ₙ-Ar oder -[C(R⁵)₂]ₙ-Het,
R⁵ H oder A',
Ar unsubstituiertes oder ein-, zwei- oder dreifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂ oder S(O)ₙA substituiertes Phenyl,
Het einen unsubstituierten oder ein- oder zweifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA und/oder Carbonylsauerstoff (=O) substituierten ein- oder zweikernigen gesättigten, ungesättigten oder aromatischen Heterocyclus mit 1 bis 4 N-, O- und/oder S- Atomen,
A' unverzweigtes oder verzweigtes Alkyl mit 1-6 C-Atomen,
A unverzweigtes, verzweigtes oder cylisches Alkyl mit 1-12 C-Atomen, worin eine oder zwei CH₂-Gruppen durch O- oder S-Atome und/oder durch -CH=CH-Gruppen und/oder auch 1-7 H-Atome durch F ersetzt sein können,
Hal F, Cl, Br oder I,
n 0, 1 oder 2,
m 0, 1, 2, 3 oder 4,
bedeuten,
sowie ihrer Salze, **dadurch gekennzeichnet, daß** man
a) eine Verbindung der Formel II
X-NH₂ II
worin
X die oben angegebene Bedeutung hat,
mit 5-Chlor-2,3-dihydro-[1,4]dioxin 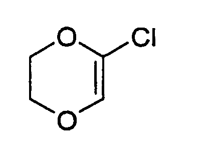 zu einer Verbindung der Formel III 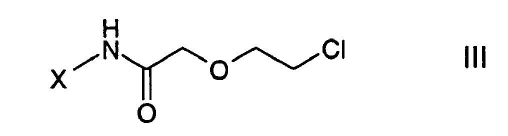 worin
X die oben angegebene Bedeutung hat,
umsetzt,
b) dann eine Verbindung der Formel III zu einer Verbindung der Formel I cyclisiert,
und
c) diese gegebenenfalls in ihr Salz überführt,
indem man eine Base oder Säure der Formel I in eines ihrer Salze umwandelt.

2. Verfahren nach Anspruch 1, zur Herstellung von Verbindungen der Formel I, worin
R¹ NO₂, CN, COOR³, COR³ oder Cl,
R² H, Hal oder A,
bedeuten,
sowie ihrer Salze.

3. Verfahren nach Anspruch 1, zur Herstellung von Verbindungen der Formel I, worin
R¹ NO₂, CN, COOR³, CON(R³)₂, COR³, SO₂R⁴, SO₂N(R³)₂, CF₃, F oder Cl,
R² H, Hal oder A,
R³ H, A, -[C(R⁵)₂]ₙ-Ar oder -[C(R⁵)₂]ₙ-Het,
bedeuten,
sowie ihrer Salze.

4. Verfahren nach Anspruch 1, 2 oder 3, zur Herstellung von Verbindungen der Formel I, worin
Ar Phenyl bedeutet,
sowie ihrer Salze.

5. Verfahren nach einem oder mehreren der Ansprüche 1-4, zur Herstellung von Verbindungen der Formel I, worin
R⁴ A bedeutet,
sowie ihrer Salze.

6. Verfahren nach einem oder mehreren der Ansprüche 1-5, zur Herstellung von Verbindungen der Formel I, worin
R¹ NO₂, CN, COOR³, CON(R³)₂, COR³, CF₃, F oder Cl,
R² H, Hal oder A',
R³ H, A' oder -[C(R⁵)₂]ₙ-Ar,
Ar Phenyl,
R⁵ H oder A',
A' unverzweigtes oder verzweigtes Alkyl mit 1-6 C-Atomen,
Hal F, Cl, Br oder I,
n 0, 1 oder 2,
bedeuten,
sowie ihrer Salze.

7. Verfahren nach einem oder mehreren der Ansprüche 1-6, zur Herstellung von Verbindungen der Formel I, wobei das Amin der Formel II einen pKₐ-Wert ≤ 3 aufweist.

8. Verfahren nach einem oder mehreren der Ansprüche 1-7, wobei die Verfahrensschritte a) und b) als Eintopfreaktion durchgeführt werden.

9. Verfahren nach einem oder mehreren der Ansprüche 1-8, wobei der Verfahrensschritt a) bei einer Temperatur zwischen 0 und 150°C durchgeführt wird.

10. Verfahren nach Anspruch 9, wobei der Verfahrensschritt a) bei einer Temperatur zwischen 70 und 90 °C durchgeführt wird.

11. Verfahren nach einem oder mehreren der Ansprüche 1-10,
wobei die Cyclisierung in einem inerten Lösungsmittel oder -gemisch, in Gegenwart eines Alkali- oder Erdalkalimetall-hydroxids, -carbonats oder -bicarbonats erfolgt.

12. Verfahren nach einem oder mehreren der Ansprüche 1-11,
wobei die Cyclisierung in Gegenwart von Cäsium- oder Kaliumcarbonat erfolgt.

13. Verfahren nach einem oder mehreren der Ansprüche 1-12,
wobei das Verfahren als Eintopfreaktion in Acetonitril erfolgt.

14. Verfahren nach einem oder mehreren der Ansprüche 1-13, zur Herstellung von Verbindungen ausgewählt aus der Gruppe
4-(4-Nitrophenyl)-3-oxo-morpholin,
4-(3-Nitrophenyl)-3-oxo-morpholin,
4-(2-Nitrophenyl)-3-oxo-morpholin,
2-Methyl-4-(4-nitrophenyl)-3-oxo-morpholin,
4-(4-Methoxycarbonylphenyl)-3-oxo-morpholin,
4-(4-Benzoylphenyl)-3-oxo-morpholin,
sowie ihrer Salze.

15. Verbindungen der Formel III 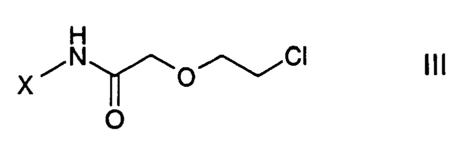 worin
X 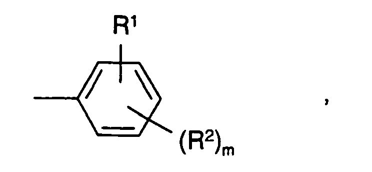
R¹ NO₂ oder CN,
R² H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-Cycloalkyl, COR³, SO₂N(R³)₂ oder SO₂R⁴,
R³ H, A, -[C(R⁵)₂]ₙ-Ar oder -[C(R⁵)₂]ₙ-Het,
R⁴ A, -[C(R⁵)₂]ₙ-Ar oder -[C(R⁵)₂]ₙ-Het,
R⁵ H oder A',
Ar unsubstituiertes oder ein-, zwei- oder dreifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂ oder S(O)ₙA substituiertes Phenyl,
Het einen unsubstituierten oder ein- oder zweifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA und/oder Carbonylsauerstoff (=O) substituierten ein- oder zweikernigen gesättigten, ungesättigten oder aromatischen Heterocyclus mit 1 bis 4 N-, O- und/oder S- Atomen,
A' unverzweigtes oder verzweigtes Alkyl mit 1-6 C-Atomen,
A unverzweigtes, verzweigtes oder cylisches Alkyl mit 1-12 C-Atomen, worin eine oder zwei CH₂-Gruppen durch O- oder S-Atome und/oder durch -CH=CH-Gruppen und/oder auch 1-7 H-Atome durch F ersetzt sein können,
Hal F, Cl, Br oder I,
n 0, 1 oder 2,
m 0, 1, 2, 3 oder 4,
bedeuten,
sowie ihre Salze.

16. Verbindungen nach Anspruch 15, worin
R¹ NO₂ oder CN,
R² H, Hal oder A,
bedeuten,
sowie ihre Salze.

17. Verbindungen nach Anspruch 15, worin
R¹ NO₂ oder CN,
R² H, Hal oder A,
R³ H, A, -[C(R⁵)₂]ₙ-Ar oder -[C(R⁵)₂]ₙ-Het,
bedeuten,
sowie ihre Salze.

18. Verbindungen nach Anspruch 15, 16 oder 17, worin
Ar Phenyl bedeutet,
sowie ihre Salze.

19. Verbindungen nach einem oder mehreren der Ansprüche 15-18, worin
R⁴ A bedeutet,
sowie ihrer Salze.

20. Verbingungen nach einem oder mehreren der Ansprüche 15-19, worin
R¹ NO₂ oder CN,
R² H, Hal oder A',
R³ H, A' oder -[C(R⁵)₂]ₙ-Ar,
Ar Phenyl,
R⁵ H oder A',
A' unverzweigtes oder verzweigtes Alkyl mit 1-6 C-Atomen,
Hal F, Cl, Br oder I,
n 0, 1 oder 2,
m 0, 1 oder 2,
bedeuten,
sowie ihre Salze.

21. Verbingungen nach Anspruch 20, worin
R¹ NO₂,
R² H, Hal oder A',
R³ H, A' oder -[C(R⁵)₂]ₙ-Ar,
Ar Phenyl,
R⁵ H oder A',
A' unverzweigtes oder verzweigtes Alkyl mit 1-6 C-Atomen,
Hal F, Cl, Br oder I,
n 0, 1 oder 2,
m 0, 1 oder 2,
bedeuten,
sowie ihre Salze.

22. Verfahren zur Herstellung von Verbindungen der Formel III, 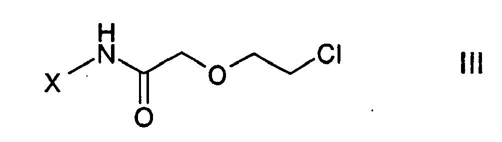 worin
x 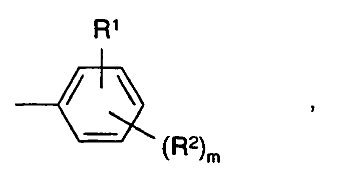
R¹ NO₂, CN, COOR³, CON(R³)₂, COR³, SO₂R⁴, SO₂N(R³)₂, CF₃, F oder Cl,
R² H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-Cycloalkyl, COR³, SO₂N(R³)₂ oder SO₂R⁴,
R³ H, A, -[C(R⁵)₂]ₙ-Ar oder -[C(R⁵)₂]ₙ-Het,
R⁴ A, -[C(R⁵)₂]ₙ-Ar oder -[C(R⁵)₂]ₙ-Het,
R⁵ H oder A',
Ar unsubstituiertes oder ein-, zwei- oder dreifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂ oder S(O)ₙA substituiertes Phenyl,
Het einen unsubstituierten oder ein- oder zweifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA und/oder Carbonylsauerstoff (=O) substituierten ein- oder zweikernigen gesättigten, ungesättigten oder aromatischen Heterocyclus mit 1 bis 4 N-, O und/oder S- Atomen,
A' unverzweigtes oder verzweigtes Alkyl mit 1-6 C-Atomen,
A unverzweigtes, verzweigtes oder cylisches Alkyl mit 1 - 12 C-Atomen, worin eine oder zwei CH₂-Gruppen durch 0- oder S-Atome und/oder durch -CH=CH-Gruppen und/oder auch 1-7 H-Atome durch F ersetzt sein können,
Hal F, Cl, Br oder I,
n 0, 1 oder 2,
m 0, 1, 2, 3 oder 4,
bedeuten,
sowie ihrer Salze, **dadurch gekennzeichnet, daß** man
a) eine Verbindung der Formel II
X-NH₂ II
worin
X die oben angegebene Bedeutung hat,
mit 5-Chlor-2,3-dihydro-[1,4]dioxin 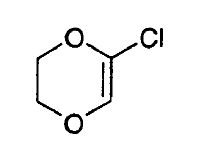 umsetzt,
und
gegebenenfalls die Verbindung der Formel III in ihr Salz überführt.

23. Verfahren nach Anspruch 22 zur Herstellung von Verbindungen der Formel III,
worin
R¹ NO₂ oder CN,
R² H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-Cycloalkyl, COR³, SO₂N(R³)₂ oder SO₂R⁴,
R³ H, A, -[C(R⁵)₂]ₙ-Ar oder -[C(R⁵)₂]ₙ-Het,
R⁴ A, -[C(R⁵)₂]ₙ-Ar oder -[C(R⁵)₂]ₙ-Het,
R⁵ H oder A',
Ar unsubstituiertes oder ein-, zwei- oder dreifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂ oder S(O)ₙA substituiertes Phenyl,
Het einen unsubstituierten oder ein- oder zweifach durch Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA und/oder Carbonylsauerstoff (=O) substituierten ein- oder zweikernigen gesättigten, ungesättigten oder aromatischen Heterocyclus mit 1 bis 4 N-, O und/oder S- Atomen,
A' unverzweigtes oder verzweigtes Alkyl mit 1-6 C-Atomen,
A unverzweigtes, verzweigtes oder cylisches Alkyl mit 1-12 C-Atomen, worin eine oder zwei CH₂-Gruppen durch 0- oder S-Atome und/oder durch -CH=CH-Gruppen und/oder auch 1-7 H-Atome durch F ersetzt sein können,
Hal F, Cl, Br oder I,
n 0, 1 oder 2,
m 0, 1, 2, 3 oder 4,
bedeuten.

24. Verfahren nach Anspruch 23 zur Herstellung von Verbindungen der Formel III,
worin
R¹ NO₂ oder CN,
R² H, Hal oder A,
bedeuten.

25. Verfahren nach Anspruch 23 zur Herstellung von Verbindungen der Formel III,
worin
R¹ NO₂ oder CN,
R² H, Hal oder A,
R³ H, A, -[C(R⁵)₂]ₙ-Ar oder -[C(R⁵)₂]ₙ-Het,
bedeuten.

26. Verfahren nach Anspruch 23 zur Herstellung von Verbindungen der Formel III,
worin
Ar Phenyl bedeutet.

27. Verfahren nach Anspruch 23 zur Herstellung von Verbindungen der Formel III,
worin
R⁴ A bedeutet.

28. Verfahren nach Anspruch 23 zur Herstellung von Verbindungen der Formel III,
worin
R¹ NO₂ oder CN,
R² H, Hal oder A',
R³ H, A' oder -(C(R⁵)₂]ₙAr,
Ar Phenyl,
R⁵ H oder A',
A' unverzweigtes oder verzweigtes Alkyl mit 1-6 C-Atomen,
Hal F, Cl, Br oder I,
n 0, 1 oder 2,
m 0, 1 oder 2
bedeuten.

## Claims

1. Process for the preparation of compounds of the formula I 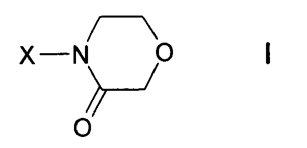 in which
X denotes 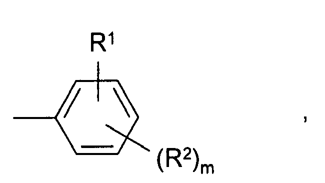
R¹ denotes NO₂, CN, COOR³, CON(R³)₂, COR³, SO₂R⁴, SO₂N(R³)₂, CF₃, F or Cl,
R² denotes H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ- cycloalkyl, COR³, SO₂N(R³)₂ or SO₂R⁴,
R³ denotes H, A, -[C(R⁵)₂]ₙ-Ar or -[C(R⁵)₂]ₙ-Het,
R⁴ denotes A, -[C(R⁵)₂]ₙ-Ar or -[C(R⁵)₂]ₙ-Het,
R⁵ denotes H or A',
Ar denotes phenyl which is unsubstituted or mono-, di- or trisubstituted by Hal, A, OR⁵, N(R⁵)₂, NO₂, CN, COOR⁵, CON(R⁵)₂, NR⁵COA, NR⁵SO₂A, COR⁵, SO₂N(R⁵)₂ or S(O)ₙA,
Het denotes a mono- or bicyclic saturated, unsaturated or aromatic heterocycle having 1 to 4 N, O and/or S atoms which is unsubstituted or mono- or disubstituted by Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA and/or carbonyl oxygen (=O),
A' denotes unbranched or branched alkyl having 1-6 C atoms,
A denotes unbranched, branched or cyclic alkyl having 1-12 C atoms, in which one or two CH₂ groups may be replaced by O or S atoms and/or by -CH=CH- groups and/or in addition 1-7 H atoms may be replaced by F,
Hal denotes F, Cl, Br or I,
n denotes 0, 1 or 2,
m denotes 0, 1, 2, 3 or 4,
and salts thereof, **characterised in that**
a) a compound of the formula II
X-NH₂ II
in which
X has the meaning indicated above,
is reacted with 5-chloro-2,3-dihydro-1,4-dioxin 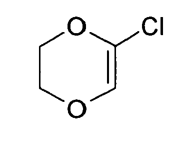 to give a compound of the formula III 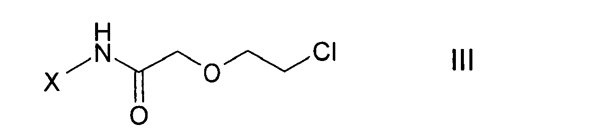 in which
X has the meaning indicated above,
b) a compound of the formula III is then cyclised to give a compound of the formula I,
and
c) this is optionally converted into its salt
by converting a base or acid of the formula I into one of its salts.

2. Process according to Claim 1 for the preparation of compounds of the formula I in which
R¹ denotes NO₂, CN, COOR³, COR³ or Cl,
R² denotes H, Hal or A,
and salts thereof.

3. Process according to Claim 1 for the preparation of compounds of the formula I in which
R¹ denotes NO₂, CN, COOR³, CON(R³)₂, COR³, SO₂R⁴, SO₂N(R³)₂, CF₃, F or Cl,
R² denotes H, Hal or A,
R³ denotes H, A, -[C(R⁵)₂]ₙ-Ar or -[C(R⁵)₂]ₙ-Het,
and salts thereof.

4. Process according to Claim 1, 2 or 3 for the preparation of compounds of the formula I in which
Ar denotes phenyl,
and salts thereof.

5. Process according to one or more of Claims 1-4 for the preparation of compounds of the formula I in which
R⁴ denotes A,
and salts thereof.

6. Process according to one or more of Claims 1-5 for the preparation of compounds of the formula I in which
R¹ denotes NO₂, CN, COOR³, CON(R³)₂, COR³, CF₃, F or Cl,
R² denotes H, Hal or A',
R³ denotes H, A' or -[C(R⁵)₂]ₙ-Ar,
Ar denotes phenyl,
R⁵ denotes H or A',
A' denotes unbranched or branched alkyl having 1-6 C atoms,
Hal denotes F, Cl, Br or I,
n denotes 0, 1 or 2,
and salts thereof.

7. Process according to one or more of Claims 1-6 for the preparation of compounds of the formula I, in which the amine of the formula II has a pKₐ value ≤ 3.

8. Process according to one or more of Claims 1-7, in which process steps a) and b) are carried out as a one-pot reaction.

9. Process according to one or more of Claims 1-8, in which process step a) is carried out at a temperature between 0 and 150°C.

10. Process according to Claim 9, in which process step a) is carried out at a temperature between 70 and 90°C.

11. Process according to one or more of Claims 1-10, in which the cyclisation is carried out in an inert solvent or solvent mixture, in the presence of an alkali or alkaline earth metal hydroxide, carbonate or bicarbonate.

12. Process according to one or more of Claims 1-11, in which the cyclisation is carried out in the presence of caesium carbonate or potassium carbonate.

13. Process according to one or more of Claims 1-12, in which the process is carried out as a one-pot reaction in acetonitrile.

14. Process according to one or more of Claims 1-13 for the preparation of compounds selected from the group
4-(4-nitrophenyl)-3-oxomorpholine,
4-(3-nitrophenyl)-3-oxomorpholine,
4-(2-nitrophenyl)-3-oxomorpholine,
2-methyl-4-(4-nitrophenyl)-3-oxomorpholine,
4-(4-methoxycarbonylphenyl)-3-oxomorpholine,
4-(4-benzoylphenyl)-3-oxomorpholine,
and salts thereof.

15. Compounds of the formula III 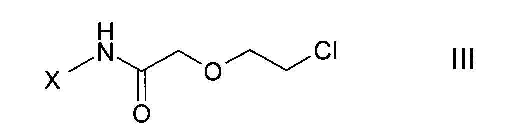 in which
X denotes 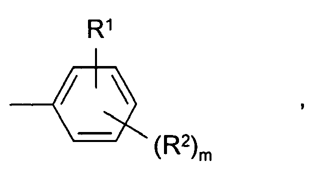
R¹ denotes NO₂ or CN,
R² denotes H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ₋cycloalkyl, COR³, SO₂N(R³)₂ or SO₂R⁴,
R³ denotes H, A, -[C(R⁵)₂]ₙ-Ar or -[C(R⁵)₂]ₙ-Het,
R⁴ denotes A, -[C(R⁵)₂]ₙ-Ar or -[C(R⁵)₂]ₙ-Het,
R⁵ denotes H or A',
Ar denotes phenyl which is unsubstituted or mono-, di- or trisubstituted by Hal, A, OR⁵, N(R⁵)₂, NO₂, CN, COOR⁵, CON(R⁵)₂, NR⁵COA, NR⁵SO₂A, COR⁵, SO₂N(R⁵)₂ or S(O)ₙA,
Het denotes a mono- or bicyclic saturated, unsaturated or aromatic heterocycle having 1 to 4 N, O and/or S atoms which is unsubstituted or mono- or disubstituted by Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA and/or carbonyl oxygen (=O),
A' denotes unbranched or branched alkyl having 1-6 C atoms,
A denotes unbranched, branched or cyclic alkyl having 1-12 C atoms, in which one or two CH₂ groups may be replaced by O or S atoms and/or by -CH=CH- groups and/or in addition 1-7 H atoms may be replaced by F,
Hal denotes F, Cl, Br or I,
n denotes 0, 1 or 2,
m denotes 0, 1, 2, 3 or 4,
and salts thereof.

16. Compounds according to Claim 15 in which
R¹ denotes NO₂ or CN,
R² denotes H, Hal or A,
and salts thereof.

17. Compounds according to Claim 15, in which
R¹ denotes NO₂ or CN,
R² denotes H, Hal or A,
R³ denotes H, A, -[C(R⁵)₂]ₙ-Ar or -[C(R⁵)₂]ₙ-Het,
and salts thereof.

18. Compounds according to Claim 15, 16 or 17 in which
Ar denotes phenyl,
and salts thereof.

19. Compounds according to one or more of Claims 15-18 in which
R⁴ denotes A,
and salts thereof.

20. Compounds according to one or more of Claims 15-19 in which
R¹ denotes NO₂ or CN,
R² denotes H, Hal or A',
R³ denotes H, A' or -[C(R⁵)₂]ₙ-Ar,
Ar denotes phenyl,
R⁵ denotes H or A',
A' denotes unbranched or branched alkyl having 1-6 C atoms,
Hal denotes F, Cl, Br or I,
n denotes 0, 1 or 2,
m denotes 0, 1 or 2,
and salts thereof.

21. Compounds according to Claim 20 in which
R¹ denotes NO₂,
R² denotes H, Hal or A',
R³ denotes H, A' or -[C(R⁵)₂]ₙ-Ar,
Ar denotes phenyl,
R⁵ denotes H or A',
A' denotes unbranched or branched alkyl having 1-6 C atoms,
Hal denotes F, Cl, Br or I,
n denotes 0, 1 or 2,
m denotes 0, 1 or 2,
and salts thereof.

22. Process for the preparation of compounds of the formula III 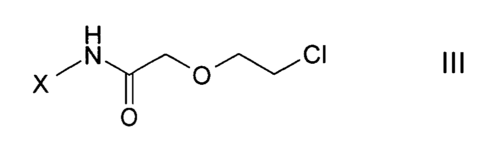 in which
X denotes 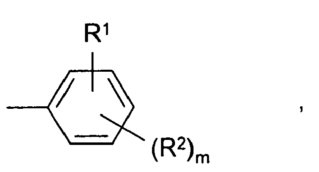
R¹ denotes NO₂, CN, COOR³, CON(R³)₂, COR³, SO₂R⁴, SO₂N(R³)₂, CF₃, F or Cl,
R² denotes H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ₋cycloalkyl, COR³, SO₂N(R³)₂ or SO₂R⁴,
R³ denotes H, A, -[C(R⁵)₂]ₙ-Ar or -[C(R⁵)₂]ₙ-Het,
R⁴ denotes A, -[C(R⁵)₂]ₙ-Ar or -[C(R⁵)₂]ₙ-Het,
R⁵ denotes H or A',
Ar denotes phenyl which is unsubstituted or mono-, di- or trisubstituted by Hal, A, OR⁵, N(R⁵)₂, NO₂, CN, COOR⁵, CON(R⁵)₂, NR⁵COA, NR⁵SO₂A, COR⁵, SO₂N(R⁵)₂ or S(O)ₙA,
Het denotes a mono- or bicyclic saturated, unsaturated or aromatic heterocycle having 1 to 4 N, O and/or S atoms which is unsubstituted or mono- or disubstituted by Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA and/or carbonyl oxygen (=O),
A' denotes unbranched or branched alkyl having 1-6 C atoms,
A denotes unbranched, branched or cyclic alkyl having 1-12 C atoms, in which one or two CH₂ groups may be replaced by O or S atoms and/or by -CH=CH- groups and/or in addition 1-7 H atoms may be replaced by F,
Hal denotes F, Cl, Br or I,
n denotes 0, 1 or 2,
m denotes 0, 1, 2, 3 or 4,
and salts thereof, **characterised in that**
a) a compound of the formula II
X-NH₂ II
in which
X has the meaning indicated above,
is reacted with 5-chloro-2,3-dihydro-1,4-dioxin 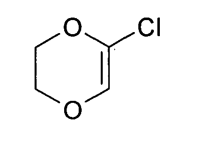 and
the compound of the formula III is optionally converted into its salt.

23. Process according to Claim 22 for the preparation of compounds of the formula III
in which
R¹ denotes NO₂ or CN,
R² denotes H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-cycloalkyl, COR³, SO₂N(R³)₂ or SO₂R⁴,
R³ denotes H, A, -[C(R⁵)₂]ₙ-Ar or -[C(R⁵)₂]ₙ-Het,
R⁴ denotes A, -[C(R⁵)₂]ₙ-Ar or -[C(R⁵)₂]ₙ-Het,
R⁵ denotes H or A',
Ar denotes phenyl which is unsubstituted or mono-, di- or trisubstituted by Hal, A, OR⁵, N(R⁵)₂, NO₂, CN, COOR⁵, CON(R⁵)₂, NR⁵COA, NR⁵SO₂A, COR⁵, SO₂N(R⁵)₂ or S(O)ₙA,
Het denotes a mono- or bicyclic saturated, unsaturated or aromatic heterocycle having 1 to 4 N, O and/or S atoms which is unsubstituted or mono- or disubstituted by Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA and/or carbonyl oxygen (=O),
A' denotes unbranched or branched alkyl having 1-6 C atoms,
A denotes unbranched, branched or cyclic alkyl having 1-12 C atoms, in which one or two CH₂ groups may be replaced by O or S atoms and/or by -CH=CH- groups and/or in addition 1-7 H atoms may be replaced by F,
Hal denotes F, Cl, Br or I,
n denotes 0, 1 or 2,
m denotes 0, 1, 2, 3 or 4.

24. Process according to Claim 23 for the preparation of compounds of the formula III
in which
R¹ denotes NO₂ or CN,
R² denotes H, Hal or A.

25. Process according to Claim 23 for the preparation of compounds of the formula III
in which
R¹ denotes NO₂ or CN,
R² denotes H, Hal or A,
R³ denotes H, A, -[C(R⁵)₂]ₙ-Ar or -[C(R⁵)₂]ₙ-Het.

26. Process according to Claim 23 for the preparation of compounds of the formula III
in which
Ar denotes phenyl.

27. Process according to Claim 23 for the preparation of compounds of the formula III
in which
R⁴ denotes A.

28. Process according to Claim 23 for the preparation of compounds of the formula III
in which
R¹ denotes NO₂ or CN,
R² denotes H, Hal or A',
R³ denotes H, A' or -[C(R⁵)₂]ₙ-Ar,
Ar denotes phenyl,
R⁵ denotes H or A',
A' denotes unbranched or branched alkyl having 1-6 C atoms,
Hal denotes F, Cl, Br or I,
n denotes 0, 1 or 2,
m denotes 0, 1 or 2.

## Revendications

1. Procédé pour la préparation de composés de la formule I 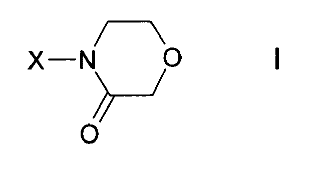 dans laquelle
X représente 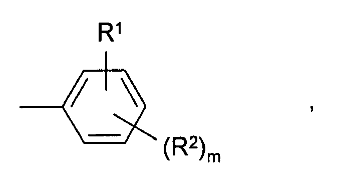
R¹ représente NO₂, CN, COOR³, CON(R³)₂, COR³, SO₂R⁴, SO₂N(R³)₂, CF₃, F ou Cl,
R² représente H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-cycloalkyle, COR³, SO₂N(R³)₂ ou SO₂R⁴,
R³ représente H, A, -[C(R⁵)₂]ₙ-Ar ou -[C(R⁵)₂]ₙ-Het,
R⁴ représente A, -[C(R⁵)₂]ₙ-Ar ou -[C(R⁵)₂]ₙ-Het,
R⁵ représente H ou A',
Ar représente phényle qui est non substitué ou mono-, di- ou trisubstitué par Hal, A, OR⁵, N(R⁵)₂, NO₂, CN, COOR⁵, CON(R⁵)₂, NR⁵COA, NR⁵SO₂A, COR⁵, SO₂N(R⁵)₂ ou S(O)ₙA,
Het représente un hétérocycle mono- ou bicyclique saturé, non saturé ou aromatique comportant de 1 à 4 atomes de N, de O et/ou de S, lequel est non substitué ou mono- ou disubstitué par Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA et/ou oxygène carbonyle (=O),
A' représente alkyle non ramifié ou ramifié comportant 1-6 atomes de C,
A représente alkyle non ramifié, ramifié ou cyclique comportant 1-12 atomes de C, où un ou deux groupes CH₂ peuvent être remplacés par des atomes de O ou de S et/ou par des groupes -CH=CH et/ou, en plus, 1-7 atomes de H peuvent être remplacés par F,
Hal représente F, Cl, Br ou I,
n représente 0, 1 ou 2,
m représente 0, 1, 2, 3 ou 4, et de leurs sels, **caractérisé en ce que**
a) un composé de la formule II
X-NH₂ II
dans laquelle
X présente la signification indiquée ci-avant,
est amené à réagir avec 5-chloro-2,3-dihydro-1,4-dioxine 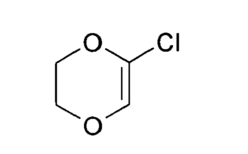 pour obtenir un composé de la formule III 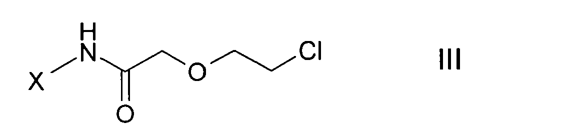 dans laquelle
X présente la signification indiquée ci-avant,
b) un composé de la formule III est ensuite cyclisé pour obtenir un composé de la formule I,
et
c) celui-ci est en option converti selon son sel
en convertissant une base ou un acide de la formule I selon l'un de ses sels.

2. Procédé selon la revendication 1 pour la préparation de composés de la formule I dans laquelle
R¹ représente NO₂, CN, COOR³, COR³ ou Cl,
R² représente H, Hal ou A,
et de leurs sels.

3. Procédé selon la revendication 1 pour la préparation de composés de la formule I dans laquelle
R¹ représente NO₂, CN, COOR³, CON(R³)₂, COR³ SO₂R⁴, SO₂N(R³)₂, CF₃, F ou Cl,
R² représente H, Hal ou A,
R³ représente H, A, -[C(R⁵)₂]ₙ-Ar ou -[C(R⁵)₂]ₙ-Het,
et de leurs sels.

4. Procédé selon la revendication 1, 2 ou 3 pour la préparation de composés de la formule I dans laquelle
Ar représente phényle,
et de leurs sels.

5. Procédé selon une ou plusieurs des revendications 1-4 pour la préparation de composés de la formule I dans laquelle
R⁴ représente A,
et de leurs sels.

6. Procédé selon une ou plusieurs des revendications 1-5 pour la préparation de composés de la formule I dans laquelle
R¹ représente NO₂, CN, COOR³ CON(R³)₂, COR³, CF₃, F ou Cl,
R² représente H, Hal ou A',
R³ représente H, A' ou -[C(R⁵)₂]ₙ-Ar,
Ar représente phényle,
R⁵ représente H ou A',
A' représente alkyle non ramifié ou ramifié comportant 1-6 atomes de C,
Hal représente F, Cl, Br ou I,
n représente 0, 1 ou 2,
et de leurs sels.

7. Procédé selon une ou plusieurs des revendications 1-6 pour la préparation de composés de la formule I, dans lequel l'amine de la formule II présente une valeur pKₐ ≤ 3.

8. Procédé selon une ou plusieurs des revendications 1-7, procédé selon lequel les étapes a) et b) sont mises en oeuvre en tant que réaction monocellulaire.

9. Procédé selon une ou plusieurs des revendications 1-8, procédé selon lequel l'étape a) est mise en oeuvre à une température entre 0 et 150°C.

10. Procédé selon la revendication 9, procédé selon lequel l'étape a) est mise en oeuvre à une température entre 70 et 90°C.

11. Procédé selon une ou plusieurs des revendications 1-10, dans lequel la cyclisation est mise en oeuvre dans un solvant inerte ou un mélange de solvants, en présence d'un hydroxyde de métaux alcalins ou de métaux alcalino-terreux, de carbonate ou de bicarbonate.

12. Procédé selon une ou plusieurs des revendications 1-11, dans lequel la cyclisation est mise en oeuvre en présence de carbonate de césium ou de carbonate de potassium.

13. Procédé selon une ou plusieurs des revendications 1-12, dans lequel le procédé est mis en oeuvre en tant que réaction monocellulaire dans de l'acétonitrile.

14. Procédé selon une ou plusieurs des revendications 1-13 pour la préparation de composés choisis parmi le groupe
4-(4-nitrophényl)-3-oxomorpholine,
4-(3-nitrophényl)-3-oxomorpholine,
4-(2-nitrophényl)-3-oxomorpholine,
2-méthyl-4-(4-nitrophényl)-3-oxomorpholine,
4-(4-méthoxycarbonylphényl)-3-oxomorpholine,
4-(4-benzoylphényl)-3-oxomorpholine,
et de leurs sels.

15. Composés de la formule III 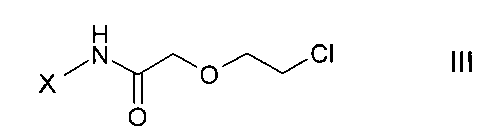 dans laquelle
X représente 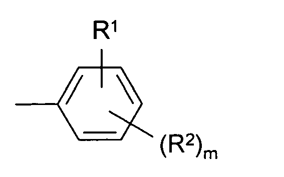
R¹ représente NO₂ ou CN,
R² représente H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-cycloalkyle, COR³, SO₂N(R³)₂ ou SO₂R⁴,
R³ représente H, A, -[C(R⁵)₂]ₙ-Ar ou -[C(R⁵)₂]ₙ-Het,
R⁴ représente A, -[C(R⁵)₂]ₙ-Ar ou -[C(R⁵)₂]ₙ-Het,
R⁵ représente H ou A',
Ar représente phényle qui est non substitué ou mono-, di- ou trisubstitué par Hal, A, OR⁵, N(R⁵)₂, NO₂, CN, COOR⁵, CON(R⁵)₂, NR⁵COA, NR⁵SO₂A, COR⁵, SO₂N(R⁵)₂ ou S(O)ₙA,
Het représente un hétérocycle mono- ou bicyclique saturé, non saturé ou aromatique comportant de 1 à 4 atomes de N, de O et/ou S qui est non substitué ou mono- ou disubstitué par Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA et/ou oxygène carbonyle (=O),
A' représente alkyle non ramifié ou ramifié comportant 1-6 atomes de C,
A représente alkyle cyclique non ramifié, ramifié ou cyclique comportant 1-12 atomes de C, où un ou deux groupes CH₂ peuvent être remplacés par des atomes de O ou de S et/ou par des groupes -CH=CH- et/ou en plus, 1-7 atomes de H peuvent être remplacés par F,
Hal représente F, Cl, Br ou I,
n représente 0, 1 ou 2,
m représente 0, 1, 2, 3 ou 4,
et leurs sels.

16. Composés selon la revendication 15, où
R¹ représente NO₂ ou CN,
R² représente H, Hal ou A,
et leurs sels.

17. Composés selon la revendication 15, où
R¹ représente NO₂ ou CN,
R² représente H, Hal ou A,
R³ représente H, A, -[C(R⁵)₂]ₙ-Ar ou -[C(R⁵)₂]ₙ-Het,
et leurs sels.

18. Composés selon la revendication 15, 16 ou 17, où
Ar représente phényle,
et leurs sels.

19. Composés selon une ou plusieurs des revendications 15-18, où
R⁴ représente A,
et leurs sels.

20. Composés selon une ou plusieurs des revendications 15-19, où
R¹ représente NO₂ ou CN,
R² représente H, Hal ou A',
R³ représente H, A' ou -[C(R⁵)₂]ₙ-Ar,
Ar représente phényle,
R⁵ représente H ou A',
A' représente alkyle non ramifié ou ramifié comportant 1-6 atomes de C,
Hal représente F, Cl, Br ou I,
n représente 0, 1 ou 2,
m représente 0, 1 ou 2,
et leurs sels.

21. Composés selon la revendication 20, où
R¹ représente NO₂,
R² représente H, Hal ou A',
R³ représente H, A' ou -[C(R⁵)₂]ₙ-Ar,
Ar représente phényle,
R⁵ représente H ou A',
A' représente alkyle non ramifié ou ramifié comportant 1-6 atomes de C,
Hal représente F, Cl, Br ou I,
n représente 0, 1 ou 2,
m représente 0, 1 ou 2,
et leurs sels.

22. Procédé pour la préparation de composés de la formule III 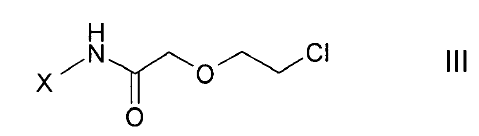 dans laquelle
X représente 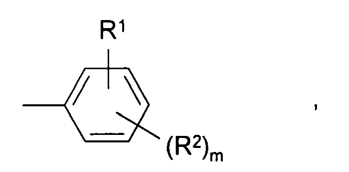
R¹ représente NO₂, CN, COOR³, CON(R³)₂, COR³, SO₂R⁴, SO₂N(R³)₂, CF₃, F ou Cl,
R² représente H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-cycloalkyle, COR³, SO₂N(R³)₂ ou SO₂R⁴,
R³ représente H, A, -[C(R⁵)₂]ₙ-Ar ou -[C(R⁵)₂]ₙ-Het,
R⁴ représente A, -[C(R⁵)₂]ₙ-Ar ou -[C(R⁵)₂]ₙ-Het,
R⁵ représente H ou A',
Ar représente phényle qui est non substitué ou mono-, di- ou trisubstitué par Hal, A, OR⁵, N(R⁵)₂, NO₂, CN, COOR⁵, CON(R⁵)₂, NR⁵COA, NR⁵SO₂A, COR⁵, SO₂N(R⁵)₂ ou S(O)ₙA,
Het représente un hétérocycle mono- ou bicyclique saturé, non saturé ou aromatique comportant de 1 à 4 atomes de N, de O et/ou de S, lequel est non substitué ou mono- ou disubstitué par Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA et/ou oxygène carbonyle (=O),
A' représente alkyle non ramifié ou ramifié comportant 1-6 atomes de C,
A représente alkyle non ramifié, ramifié ou cyclique comportant 1-12 atomes de C, où un ou deux groupes CH₂ peuvent être remplacés par des atomes de O ou de S et/ou par des groupes -CH=CH- et/ou, en plus, 1-7 atomes de H peuvent être remplacés par F,
Hal représente F, CI, Br ou I,
n représente 0, 1 ou 2,
m représente 0, 1, 2, 3 ou 4,
et de leurs sels, **caractérisé en ce que**
a) un composé de la formule II
X-NH₂ II
dans laquelle
X présente la signification indiquée ci-avant,
est amené à réagir avec 5-chloro-2,3-dihydro-1,4-dioxine 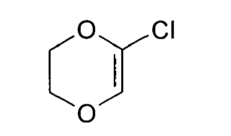 et
le composé de la formule III est en option converti selon son sel.

23. Procédé selon la revendication 22 pour la préparation de composés de la formule III
dans laquelle
R¹ représente NO₂ ou CN,
R² représente H, Hal, A, OR³, N(R³)₂, NO₂, CN, COOR³, CON(R³)₂, NR³COA, NR³CON(R³)₂, NR³COOR³, NR³SO₂A, -[C(R⁵)₂]ₙ-Ar, -[C(R⁵)₂]ₙ-Het, -[C(R⁵)₂]ₙ-cycloalkyle, COR³, SO₂N(R³)₂ ou SO₂R⁴,
R³ représente H, A, -[C(R⁵)₂]ₙ-Ar ou -[C(R⁵)₂]ₙ-Het,
R⁴ représente A, -[C(R⁵)₂]ₙ-Ar ou -[C(R⁵)₂]ₙ-Het,
R⁵ représente H ou A',
Ar représente phényle qui est non substitué ou mono-, di- ou tri- substitué par Hal, A, OR⁵, N(R⁵)₂, NO₂, CN, COOR⁵, CON(R⁵)₂, NR⁵COA, NR⁵SO₂A, COR⁵, SO₂N(R⁵)₂ ou S(O)ₙA,
Het représente un hétérocycle mono- ou bicyclique saturé, non saturé ou aromatique comportant de 1 à 4 atomes de N, de O et/ou de S, lequel est non substitué ou mono- ou disubstitué par Hal, A, OR⁵, N(R⁵)₂, NO₂, CN, COOR⁵, CON(R⁵)₂, NR⁵COA, NR⁵SO₂A, COR⁵, SO₂N(R⁵)₂, S(O)ₙA et/ou oxygène carbonyle (=O),
A' représente alkyle non ramifié ou ramifié comportant 1-6 atomes de C,
A représente alkyle non ramifié, ramifié ou cyclique comportant 1-12 atomes de C, où un ou deux groupes CH₂ peuvent être remplacés par des atomes de O ou de S et/ou par des groupes -CH=CH- et/ou, en plus, 1-7 atomes de H peuvent être remplacés par F,
Hal représente F, Cl, Br ou I,
n représente 0, 1 ou 2,
m représente 0, 1, 2, 3 ou 4.

24. Procédé selon la revendication 23 pour la préparation de composés de la formule III
dans laquelle
R¹ représente NO₂ ou CN,
R² représente H, Hal ou A.

25. Procédé selon la revendication 23 pour la préparation de composés de la formule III
dans laquelle
R¹ représente NO₂ ou CN,
R² représente H, Hal ou A,
R³ représente H, A, -[C(R⁵)₂]ₙ-Ar ou -[C(R⁵)₂]ₙ-Het.

26. Procédé selon la revendication 23 pour la préparation de composés de la formule III
dans laquelle
Ar représente phényle.

27. Procédé selon la revendication 23 pour la préparation de composés de la formule III
dans laquelle
R⁴ représente A.

28. Procédé selon la revendication 23 pour la préparation de composés de la formule III
dans laquelle
R¹ représente NO₂ ou CN,
R² représente H, Hal ou A',
R³ représente H, A' ou -[C(R⁵)₂]ₙ-Ar,
Ar représente phényle,
R⁵ représente H ou A',
A' représente alkyle non ramifié ou ramifié comportant 1-6 atomes de C,
Hal représente F, Cl, Br ou 1,
n représente 0, 1 ou 2,
m représente 0, 1 ou 2.
